# EUROPEAN PATENT APPLICATION

(11) **EP 3 594 338 A1**
(43) Date of publication of application: **15.01.2020**
(21) Application number: 18183258.5
(22) Date of filing: 12.07.2018
(51) Int. Cl.: C12N 15/10, B03D 3/06, C12N 1/06, B01F 13/02, C12M 1/04

(54) **METHOD AND APPARATUS FOR THE PURIFICATION OF EXTRA-CHROMOSOMAL NUCLEIC ACID SEQUENCES**

(71) Applicant: Kaneka Eurogentec SA, 4102 Seraing (BE)
(72) Inventor: JOST, Laurent, 4102 Seraing (BE)
(74) Representative: Pronovem

(57) **Abstract**

The present invention relates to a new apparatus and a new method for obtaining the recovery of pre-purified extra-chromosomal nucleic acid sequences.

## Description

### Field of the invention

The present invention is in the field of biotechnology and concerns a method and an apparatus for the purification from contaminants and impurities of polynucleotide sequences, preferably extra-chromosomal nucleic acid sequence(s) selected from the group consisting of DNA plasmids, cosmids, BACs, YACS, MACs, mini-plasmids and mini-circles.

### Background of the invention and state of the Art

The purification of polynucleotides is generally obtained from host cells able to produce large quantities of these polynucleotides, possibly after genetic engineering.

It is known that a biomass of gram-negative bacteria, such as *Escherichia coli* can be lysed and its nucleotides sequences of interest, especially extra-chromosomal nucleic acid sequences, separated from the bulk of genomic nucleic acids and proteins by successive steps including a selective precipitation, a sedimentation, a filtration, and a specific retention on chromatographic columns.

Depending on the protocol followed, these nucleic acids sequences of interest are combined with contaminants (or impurities), such as endotoxins, phenols, caesium chlorides, ethidium bromides, Triton®, bound proteins or other nucleic acids.

As highly-purified nucleotides sequences; especially extra-chromosomal nucleic acid sequences are required for specific uses in laboratories or for clinical purposes, several attempts to reduce the amount of contaminants were proposed.

Generally, methods applied for their isolation involve a lysis step of cells with the addition of alkali or enzymes, before a mixing with a neutralizing solution and eventually a precipitating solution to recover the extra-chromosomal sequence(s) in solution from precipitated contaminating cell components (genomic DNA, proteins and protein-nucleic complexes).

The use of alkaline solution, heating step and/or long procedure will degrade these nucleotide sequences. In addition, DNA molecules, including these extra-chromosomal nucleic acid sequence(s) are sensitive to mechanical stress. Furthermore, highly viscous solution may cause either local heterogeneities or require extensive mixing, both having the potential to degrade these nucleotide sequences. This is especially the case when concentrated chloride of divalent metals (such as CaCl₂) solutions are used.

It is known that batch wise methods present a risk of contamination and/or heterogeneity.

The document WO2010/0136503 describes a method and an apparatus for an efficient purification of a DNA plasmid, wherein the precipitation of the DNA plasmid contaminants is obtained by a mixing of the disintegrated cells with a solution containing one or more salts, such as CaCl₂, in a passageway and by a subsequent separation of the precipitates from the solution through gravimetric decantation.

In this method and apparatus, an efficient mixing of the disintegrated cells and salts and the subsequent precipitation is obtained by the integration of means inducing a Venturi effect in the tubing elements. This Venturi effect is caused by a reduction in the internal diameter in the tubing elements of about 40%.By the terms "inducing a Venturi effect", it was meant a turbulence obtained from a system, wherein a fluid in laminar flow is forced to pass into reduced tubing in such an extent that the fluid has an increased speed and that a depression is caused just after the reduced diameter.

A created Venturi effect in the tubing elements causes an efficient mixing obtained without degrading the DNA plasmid and without creating high shearing forces in the liquid. Therefore, no degradation of DNA molecules that is known to occur under heavy stirring or heavy shearing forces, which is necessary given the high viscosity of a 5 M CaCl₂ solution was observed in the described method. However, such method and apparatus requiring integration of means for inducing a Venturi effect, is dedicated to the production of few grams of plasmid and is not adequate for a production of larger amounts of extra-chromosomal nucleic acid sequences.

The documents EP 811055-B1, WO99/37750 and WO00/05358 describe methods and apparatus of cellular lysis wherein a mixing of cells with a lysing solution is done into thin tubings or static mixers having an adequate diameter and/or length to obtain such lysis.

Furthermore, various means and steps were described in the scientific literature to improve the yield and efficiency of the purification steps of nucleic acids extracted from cells. For instance, the patent US 5,096,818-B2 discloses a method for isolating and purifying nucleic acid from a cells culture media by adding to resuspended cells, the lysing/denaturing and the deproteinating and/or neutralizing agents without mixing, followed by a first centrifugation that partially pellets the cellular debris, and then mixing the solution to finalize the lysis, followed by a second centrifugation to completely pellet the cellular debris. This centrifugation method is not suitable for large scales due to equipment constraints.

The European patent EP 1 593 741-B1 discloses a method for producing a microbial clear lysate containing extra-chromosomal nucleic acid sequence(s) by mixing a suspension of microorganisms containing these nucleic acid sequences with a reaction buffer, reacting the mixture for a duration sufficient to break the microorganisms, transferring the obtained mixture into a vessel comprising a neutralizing buffer to form a precipitate with the contaminants to remove and generating gas bubbles by dispersing a gas through the bottom of the vessel to separate the precipitate from the lysate. As the neutralization buffer is not added in continuous but is present from the beginning of the lysis, its concentration (and therefore its neutralization power) changes along the time, which means process variability. It also means that very large quantities of cells cannot be treated easily due to the system design.

### Aims of the invention

The present invention is related to a method and an apparatus which do not present the drawbacks of the methods and apparatus of the state of the art.

A preferred aim of the present invention is to obtain a cheap, robust and simplified method and apparatus for an efficient and rapid purification, especially through an improved clarification step, of extra-chromosomal nucleic acids sequences of interest, especially a purification of large amounts of these extra-chromosomal nucleic acids sequences of interest, in particular in amounts that are larger than the amount (s) obtained with the methods and apparatus of the state of the art.

### Summary of the invention

In the method and apparatus according to the invention, by the terms "about" or "around", it is preferably meant a value plus or minus 20 or 10% (e.g. about 5 minutes means every values from 4 minutes and 30 seconds to 5 minutes and 30 seconds) above or lower the mentioned value.

The present method for obtaining by successive production and/or purification steps one or more extra-chromosomal nucleic acid sequence(s) of interest, preferably a DNA plasmid, preferably having a size lower than 30.000 bases (or base pairs), more preferably lower than 15.000 bases, comprises (or consists of) the steps of:
a) optionally cultivating (preferably recombinant) hosts cells producing (involved in the synthesis of) the sequence(s) of interest and harvesting these cells containing the sequence(s) of interest, preferably cells containing this DNA plasmid of interest;
b) disintegrating these cells (through introduction into a passageway) in a continuous process (i.e. continuously in a continuous flow device), preferably through addition of an (alkaline) lysis medium to the cells ;
c) performing a neutralization step of the lysed cells with a neutralisation solution, preferably a neutralization solution composed of an acetic acid/acetate composition, to produce a cells lysate mixture formed by a soluble fraction and a precipitate;
d) optionally (further) precipitating contaminants or impurities of the extra-chromosomal nucleic acid sequence(s) of interest, especially of the DNA plasmid of interest, by mixing the first mixture with one or more, preferably hydrated, salt (s) selected from the group consisting of (preferably hydrated) CaCl₂, (preferably hydrated) MgCl₂, (preferably hydrated) ZnCl₂, (preferably hydrated) SrCl₂, (preferably hydrated) BaCl₂, or (less preferably) other (preferably hydrated) salt(s), such as LiCl, ammonium acetate, ammonium sulfate, sodium sulfate or magnesium sulfate, wherein the preferred salts are (preferably hydrated) CaCl₂ and/or (preferably hydrated) MgCl₂ that is (are) preferably added (in continuous) to these disintegrated cells in a solution at a concentration comprised between (about) 2M and (about) 6 M); step (d) being carried out to advantageously purify the cells lysate mixture;
e) separating the soluble fraction (comprising the extra-chromosomal nucleic acid sequence(s) of interest, especially the DNA plasmid of interest) from the precipitate (e.g., comprising contaminants or impurities) contained in the cells lysate mixture, preferably in a tank and for a period preferably comprised between (about) 5 minutes and a few hours; more preferably between (about) 15 minutes and (about) 2 hours;
f) recovering, preferably by a pumping out, the separated soluble fraction comprising the extra-chromosomal nucleic acid sequence(s) of interest, especially the DNA plasmid of interest from this cells lysate mixture. Advantageously, the precipitate comprising the contaminants or impurities is not recovered;
g) optionally performing one or more further decantation step(s) and/or one or more filtration step(s) on the soluble fraction upon one or more filter(s) having a pore size comprised between (about) 0.2 µm and (about) 20 µm, preferably followed by an ultra-filtration step upon an (about) 50 kDa membrane to (about) 500 kDa membrane, preferably between (about) 50 and (about) 250 kDa, more preferably between (about) 50 and (about) 100 kDa to obtain a first membrane retentate comprising the extra-chromosomal nucleic acid sequence(s) of interest, preferably the DNA plasmid of interest;
h) optionally performing a (polishing step) chromatography of the soluble fraction from step f) or of the first membrane retentate from step g), comprising the recovered extra-chromosomal nucleic acid sequences(s), optionally with a washing substep to obtain a chromatography eluate;
i) optionally performing an ultrafiltration of the chromatography eluate upon an about 30 kDa to about 70 kDa membrane and collecting an obtained second retentate and possibly performing a (final) filtration of the second retentate upon an about 0.2 µm membrane to recover and collect in a filtrate, the extra-chromosomal nucleic acid sequence(s) of interest, preferably the DNA plasmid of interest, purified from contaminants or impurities.

According to aspects of the present invention, the recovering step f) comprises injecting an aqueous medium comprising a dissolved gas into the cells lysate mixture. It has surprisingly been found that the injection of an aqueous medium comprising a sufficient amount of dissolved gas, which can be obtained by dissolving a gas in the aqueous medium at an elevated pressure as described herein, improves the separation between the soluble fraction comprising the extra-chromosomal nucleic acid sequences of interest and the precipitate, and therefore allows for improving the recovery of such nucleic acid sequences.

The aqueous medium with a dissolved gas is advantageously obtained by bubbling a gas, preferably a gas selected from the group consisting of air, CO₂ or nitrogen or a mixture thereof, into an aqueous medium, preferably liquid, such as water, preferably into a reservoir or tank, prior to injection of the aqueous medium into the cells lysate mixture. The gas is dissolved in the aqueous medium, e.g. by bubbling, advantageously at a pressure above atmospheric pressure, such as a pressure of at least 1 barg, preferably between 2 and 50 barg, preferably between 3 and 25 barg, preferably between 4 barg and 15 barg. By so doing, a higher concentration of dissolved gas in the aqueous medium can be obtained (Henry's law). The concentration of the dissolved gas in the aqueous medium advantageously approximates the saturation concentration, e.g. the concentration of the dissolved gas in the aqueous medium amounts to at least 50% of the saturation concentration at the indicated pressures, advantageously at least 60%, at least 70%, at least 80%, at least 90% of the saturation concentration. Advantageously, the aqueous medium is saturated with the dissolved gas. The aqueous medium is preferably saturated in gas at a pressure higher than (about) 2 barg, preferably comprised between (about) 2 barg and (about) 50 barg, more preferably between (about) 3 barg and (about) 25 barg. Advantageously, the volume amount of the aqueous medium comprising the dissolved gas injected in the cells lysate mixture compared to the volume of the cells lysate mixture being treated (e.g. total volume of the cells lysate mixture in the tank), is comprised between (about) 0.2% v/v and (about) 25% v/v, preferably between (about) 0.5% v/v and (about) 15%, more preferably between (about) 1% v/v and (about) 10%, injected once or several times.

Advantageously, the aqueous medium is injected under pressure in the cells lysate mixture the latter being advantageously at atmospheric pressure. Advantageously, the aqueous medium is injected at substantially a same pressure as the pressure at which the gas is dissolved. Preferable injection pressures are at least 2 barg, preferably between (about) 3 barg and (about) 50 barg, preferably between 4 barg and 15 barg. The aqueous medium is advantageously injected in the cells lysate mixture intermittently. Advantageously a rest period between two consecutive injection periods has at least equal duration as either injection periods. By way of example, a time period during which injection takes place is between (about) 5 seconds and (about) 10 minutes, more preferably of (about) 5 seconds and (about) 5 minutes. The rest period between two consecutive injection periods is advantageously between 5 seconds and 10 minutes, advantageously at least equal to the injection time period.

An intermittent injection of the aqueous medium comprising the dissolved gas allows the precipitate to raise and the soluble fraction to clarify during the rest time. As a result, a more clarified soluble fraction can be obtained which comprises the extra-chromosomal nucleic acid sequences of interest.

By the terms "extra-chromosomal nucleic acid sequence", it is meant any nucleotide sequence of more than 50 bases (base pairs). More preferably, this extrachromosomal nucleic acid sequence is selected from the group consisting of DNA plasmids, cosmids, BACSs, YACs, MACs, mini-plasmids and mini-circles, preferably a (DNA) plasmid, such as a (DNA) plasmid of a size smaller than 30.000 base pair.

The host cells used in the method and integrated in the apparatus or plant according to the invention are prokaryotic cells, such as bacteria, especially *E. coli cells.*

In the method according to the invention, the mixing step of step b) of cells with the (alkaline) lysis medium is done into a static mixer, preferably into a static mixer comprising at least 6 mixing elements, preferably between 12 mixing elements and 24 mixing elements, more preferably 18 mixing elements, wherein the mixing linear speed of the introduced mixture of cells and lysis medium is equal or higher than 150 cm/min; preferably comprised between (about) 150 cm/min and (about) 2000 cm/min, more preferably comprised between (about) 1000 cm/min and (about) 1500 cm/min.

In the method according to the invention, the mixing step of step d) of the cells lysate mixture obtained from step c) with the salt(s) is done into a static mixer, preferably into a static mixer comprising at least 2 mixing elements, preferably between 2 and 18 mixing elements, more preferably 4 mixing elements, wherein the mixing linear speed of the introduced mixture of lysed cells and salts is equal or higher than 100 cm/min, preferably comprised between (about) 100 cm/min and (about) 1500 cm/min, more preferably between (about) 400 cm/min and (about) 1200 cm/min.

In the method according to the invention, the mixing step of step c) of lysed cells with the neutralization solution is done into a static mixer, preferably into a static mixer comprising at least 4 mixing elements, preferably between 6 and 16 mixing elements, more preferably 10 mixing elements, wherein the mixing linear speed of the introduced lysed cells in the neutralization solution is equal or higher than 100 cm/min, preferably comprised between (about) 100 cm/min and (about) 2000 cm/min, more preferably comprised between (about) 340 cm/min and (about) 1025 cm/min.

In the method and apparatus of the invention, the cells are preferably maintained in a suspension and disintegrated by an addition of a (alkaline) lysis medium.

According to the invention, the lysis medium to disintegrate cells is an alkaline solution presenting a pH comprised between (about) 11 and (about) 12.5, preferably a pH comprised between (about) 12 and (about) 12.5 and this medium is supplemented with a sufficient amount of one or more detergent(s).

In the method and apparatus according to the invention, this (alkaline) lysis medium is resulting from a mixing of a sufficient amount of NaOH with a sufficient amount a detergent, preferably Sodium Dodecyl Sulfate (SDS). More preferably, this mixing is obtained through introduction of sufficient amounts of each reactive product solution (NaOH and the detergent) into a static mixer, before their addition to the cells. Therefore, the (alkaline) lysis medium to disintegrate the cells may further comprise between (about) 0.01 % and (about) 5%, preferably between (about) 0.1 % and (about) 3%, more preferably between (about) 0.5 % and (about) 2% (w:v) of one or more detergent(s), preferably a detergent selected from the group consisting of Sodium Dodecyl Sulfate (SDS), Sodium Deoxycholate, Triton® X-100, Triton® X-114, Nonidet® P-40, Octyl-glucoside, Brij® 35, Brij® 56 Tween® 20 and CHAPS(3-[(3-Cholamidopropyl)dimethylammonio]-1-propanesulfonate) or a mixture thereof.

In the method according to the invention, the (alkaline) lysis medium is advantageously obtained by a mixing of at least a sufficient amount of NaOH and a sufficient amount of a detergent (such as SDS) and maybe one or more of the additional components above described, into a static mixer, preferably into a static mixer comprising at least 6 mixing elements, preferably between 12 and 24 mixing elements, more preferably 18 mixing elements and wherein the mixing linear speed of these introduced elements is equal or higher than 100 cm /min.

Advantageously, the optimal contact time between the cells presented in suspension and the (alkaline) lysis medium used to disintegrate these cells, is comprised between (about) 15 seconds and (about) 15 minutes, preferably between (about) 0.5 minutes and (about) 5 minutes, more preferably of about 2 minutes.

Preferably, the neutralization solution is acetic acid/acetate solution having a pH comprised between (about) 5.0 and (about) 6.0, preferably of about 5.5 and has a concentration of about 3 M (mol/l) of acetate and about 15% (v:v) of acetic acid.

Preferably, this acetic acid/acetate solution is chilled at a lower temperature from room temperature towards a lower temperature comprised between (about) 2°C and (about) 8°C, preferably of about 4°C, by storing the buffer in a cold room for a sufficient amount of time or by using an adequate cryostat connected to a heat exchanger surrounding the tubing element carrying the neutralization solution.

Advantageously, the optimal contact time of these lysed cells comprising the extra chromosomal nucleic acid sequence(s) of interest and the neutralization solution is comprised between (about) 15 seconds and (about) 5 minutes, preferably is between (about) 30 seconds and (about) 2 minutes, more preferably of about 1 minute.

Advantageously, the optimal contact time of the neutralized lysate and the precipitation solution is higher than 1 minute, this period being suitable for obtaining the required precipitation.

Advantageously, the optimal time of step e) of the method according to the invention and related to the decantation, the sedimentation and/or the floatation is comprised between (about) 5 minutes and several hours, preferably between (about) 15 minutes and (about) 2 hours.

The method of the invention may optionally comprise a preliminary step (between step a) and step b)) comprising (or consisting of) the addition of a sufficient amount of RNase to the (whole) cells (suspension) comprising the extra-chromosomal nucleic acids sequence(s) of interest. This RNase treatment is especially useful for a purification of plasmid DNA having a size inferior to 3000 bases (base pairs).

The preferred added hydrated salt of the method according to the invention is CaCl₂.5H₂O which is added at a concentration comprised between (about) 2 M and (about) 6 M, preferably at a concentration of about 5 M.

In the method of the invention, the continuous process or the continuous addition steps are performed via an opening of inlets / outlets, pumps or valves and these pumps outputs are advantageously controlled by weighing the tanks, reservoirs, vials or recipients of the feeding solutions of the invention or by integration of flowmeters to the tubing(s) connecting these tanks.

In the method and apparatus of the invention, the filtration step is done on depth filters or on surface filters.

The chromatography step (step h) possibly present in the method and apparatus of the invention comprises (or consist of) classical purification steps well known by the person skilled in the art, preferably comprising (or consisting of) the sub-steps of:
- binding the extra-chromosomal nucleic acids sequence(s) of interest present in the first ultrafiltration retentate,
- washing and eluting the obtained extra-chromosomal nucleic acids sequence(s) of interest.

Optionally, after this washing sub-step and before the elution sub-step, another washing sub-step is performed with the same solution supplemented with one or more neutral detergent(s), preferably detergent(s) selected from the group consisting of Triton® X-100, Triton® X-114, Tween® 20, Nonidet® P-40, octylglucoside, Brij® 35, Brij® 56, or a mixture thereof, preferably present in the solution at (about) 0.1% to (about) 1%, and is followed by a third washing sub-step without neutral detergent(s).

The method and apparatus of the invention allows the production and purification of large amounts of extra-chromosomal nucleic acid sequence(s) of interest, from less than one gram to hundreds of grams or even kilograms. It will be convenient to note that the precipitation step d) is optional and may be replaced by additional purification steps downstream the process in order to obtain extra-chromosomal nucleic acid sequences of interest of desired purity.

The invention also relates to an apparatus for carrying out methods according to the present invention. The apparatus of the present invention comprises (or consists of) means for obtaining a continuous flow formed with circular tubes or tubings, possibly connected to suitable tanks, reservoir(s), vials or recipient(s) and having inlet or opening means for the introduction of media and cells or cells fractions and outlet or exit means for the recovery of extra-chromosomal nucleic acid sequence(s) of interest to be obtained from these cells and purified from contaminants or impurities.

Advantageously, the tubing (s) of the invention are in accordance to the requirements for use in human medicine, especially with Pharma quality, preferably are non-leachable and are linked through "Y-type connections.

The apparatus of the present invention comprises or preferably consists of:
- a preparation unit comprising one or more tanks containing compounds of a (alkaline) lysis medium, the tank(s) being fluidly connected, preferably via one or more tubing(s) to the inlet of a cells lysis unit
- a cells lysis unit comprising a cell suspension tank containing cells with the extra-chromosomal nucleic acid sequences of interest, an inlet and an outlet or exit, the inlet being fluidly connected, preferably via one or more tubing (s), to the cells suspension tank and to the outlet or exit of the preparation unit, and the outlet being fluidly connected to the inlet of the neutralization unit
- a neutralization unit comprising a neutralization medium tank, an inlet and an outlet or exit, the inlet being in fluid connection, preferably via one or more tubing(s), to the neutralization medium tank and to the outlet or exit of the lysis unit;
- optionally a precipitation unit comprising an inlet, an outlet or exit and a tank containing a solution of salts, the inlet being in fluid connection, preferably via one or more tubing(s), to the tank containing a solution of salts and to the outlet or exit of the neutralization unit;
- a separation unit, in particular a decantation unit, comprising a separation (decantation) tank for a cells lysate mixture, having an inlet fluidly connected to the outlet of the neutralization unit, or as the case may be, of the precipitation unit for receiving a cells lysate mixture, and preferably comprising means for recovering the soluble fraction containing the extra-chromosomal nucleic acid sequences of interest from this cells lysate mixture, this separation unit being linked, preferably via one or more tubing(s) to
- an injection unit, in particular a floatation unit, comprising means for dissolving a gas in the aqueous medium at a pressure higher than atmospheric pressure and means for injecting the aqueous medium, preferably water, preferably saturated in dissolved gas, preferably air, CO₂ or Nitrogen, or a mixture thereof, into the separation tank.

Preferably, the injection unit includes at least one injection pipe having an exit, preferably an exit nozzle oriented towards the bottom surface of the decantation tank. The exit, preferably the exit nozzle being preferably disposed in the decantation tank at a height comprised between (about) 1/6 and (about) 5/6 of the total height of the tank, more preferably between (about) 1/4 and 2/4 of the total height of the decantation tank. This position advantageously avoids the resuspension of the precipitated contaminants and impurities that already have been separated to the top of the decantation tank, in the cells lysate mixture during the aqueous medium injection. In the apparatus of the invention, the injection unit comprises a water or aqueous medium tank and means for bubbling the gas through this aqueous medium. Preferably, these means for bubbling gas are configured to inject gas in the aqueous medium tank at a pressure higher than or equal to 2 barg, preferably between 2 barg and 50 barg, preferably between 3 barg and 25 barg and advantageously in a volume comprised between (about) 5% and (about) 25% V/V compared to the volume of cells lysate mixture to treat.

In the apparatus according to the invention, the ratio between the injection pipe internal diameter and its exit nozzle diameter could be (about) 1 or near the value of 1, but is preferably higher than (about) 2, but preferably lower than 50, more preferably lower than 25 and is able to generate in the cells lysate mixture (a fog of) (micro)bubbles having an average diameter smaller than or equal to 1.5 mm, preferably smaller than or equal to 1 mm or even smaller than or equal to 0.5 mm.

The apparatus according to the invention may further include either known mixing elements, such as means generating Venturi effect (as described in the international patent application WO2010/0136503 described herein by reference), possibly combined with one or more static mixer(s) for the efficient mixing of the fluids used in the purification method according to the invention.

In the apparatus according to the invention, the preparation unit comprises either a single tank containing the lysis medium or separated tanks containing its different components connected to the cells lysis unit by a first (set of) tubing(s) via one (or several) pump(s). A first static mixer, preferably comprising at least 6 mixing elements can be provided between the separated tanks and the cells lysis unit, this first static mixer having an inlet and an outlet or exit, this inlet being connected to the tubing (s) coming from the pump(s) of the (alkaline) lysis medium or its separated components, and the outlet or exit being connected, via a second tubing, to the cells lysis unit. The size (i.e. number of introduced mixing elements, as well as the diameter and the length) of the first static mixer and the pump(s) output (s) are selected to allow a mixing linear speed of the introduced elements into the first static mixer that is preferably higher than 100 cm/min.

The apparatus according to the invention may also comprise a cells lysis unit made of a second static mixer, preferably a second static mixer comprising between 12 and 24, preferably 18 mixing elements, this second static mixer having an inlet and an outlet or exit, the inlet being connected by the second tubing to the outlet of the preparation unit and being connected via a third tubing and a pump to the cells suspension tank containing the cells suspension, and the outlet or exit being connected to a fourth tubing. The size of the second static mixer and the pumps outputs are selected to allow a mixing linear speed of the introduced solutions into the second static mixer that is comprised between (about) 1000 cm/min and (about) 1500 cm/min.

The apparatus according to the invention may further comprise a third static mixer, preferably a third static mixer comprising between 6 mixing elements and 16 mixing elements, preferably 10 mixing elements, this third static mixer having an inlet and an outlet or exit, the inlet being connected to a fourth tubing coming from the outlet of the cells lysis unit and being connected, via a fifth tubing and a pump to a neutralization medium tank, the outlet or exit being connected to a sixth tubing. The size of the third static mixer and the pumps outputs are selected to allow a mixing linear speed of the introduced solutions into the third static mixer that is preferably comprised between (about) 340 cm/min and (about) 1025 cm/min.

The apparatus according to the invention may further comprise a fourth static mixer, preferably a fourth static mixer comprising between 4 and 18 mixing elements.

This fourth static mixer having an inlet and an outlet or exit, the inlet being connected to a sixth tubing coming from the neutralization unit and connected via a seventh tubing and a pump, to a tank containing a solution of salts, and wherein the outlet or exit is connected via an eighth tubing to a decantation tank. The size of the fourth static mixer and the pumps outputs are selected to allow a mixing linear speed of the introduced solutions into the fourth static mixer that is preferably comprised between (about) 400 cm/min and (about) 1200 cm/min.

The position of the pumps as represented in the figures also improves the treatment efficiency, because the pumps respective position as represented can advantageously allow a pushing (the flow) of fluids in the tubing(s), instead of a pulling of fluids present in these tubing(s), meaning that the treated product is not impacted by the shearing forces occurring in the pumps heads that could damage it and lower its quality.

Preferably, the apparatus of the invention further comprises means to weight the feeding solutions present in the different tanks, reservoirs, bags or recipients and/or comprises one or more flowmeter (s), for controlling the introduction of the different media or cells or cells fractions in the tubing(s).

The present invention is described in reference to the enclosed figures in the following detailed description presented as a non-limiting preferred embodiment of the method and apparatus according to the present invention.

### Short description of the figures

The figure 1 represents schematically the apparatus of the invention.
The figures 2 and 3 represent different configurations of the decantation unit of the apparatus according to the invention.
The figure 4 represents the floatation device connected to the decantation unit of the apparatus according to the invention.
Figure 5 represents a filtration unit connected to the separation (decantation) unit of the apparatus according to the invention.

### Detailed description of the invention

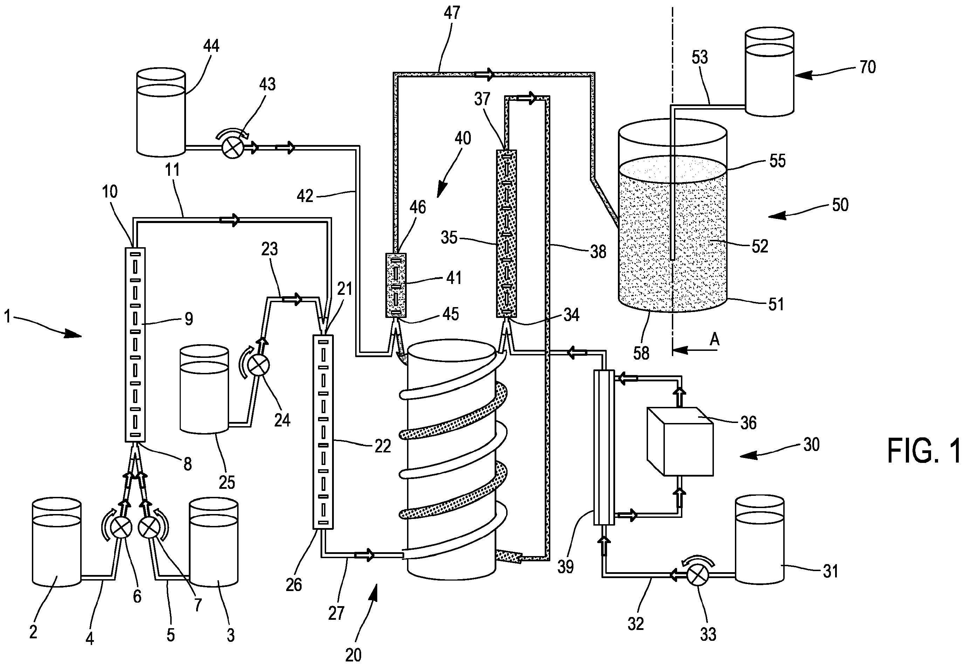

The figure 1 illustrates the apparatus allowing to carry-out steps a) to e) of the method of the present invention and being a pipe of several meters long, formed with circular tubes or tubing elements fluidly connected or linked together and connected to static mixers and tanks or reservoirs.

Preferably, the apparatus as well as the method according to the invention are dedicated to the recovery and to the purification from cells, of an extra chromosomal nucleic acid sequence, such as a DNA plasmid that will be purified from cells contaminants, especially from *E. Coli* contaminants, such as cells proteins, RNA sequences, Genomic DNA sequences, HCPs,...

The apparatus used in the method according to the invention comprises or consists of several units that are fluidly connected and include a preparation unit 1, a cells lysis unit 20, a neutralization unit 30, optionally a precipitation unit 40, a decantation unit 50, a filtration unit 60 and a floatation unit 70.

Furthermore, the cells lysis unit 20 could be fluidly connected to a production unit (not represented in the figures), but used for the growth and multiplication of cells comprising the extra-chromosomal nucleic acid sequence(s) of interest to be recovered.

In the passageway of the apparatus according to the invention the flow rates follow-up is obtained by chronometers, weight monitoring and/or by flow meters added to the different tubing(s) and connections between tubing(s) are of the "Y type"

The first part of the apparatus according to the invention, is a preparation unit 1 which can present two different formats. According to a first embodiment, not presented in the figures, the apparatus according to the invention contains a tank or reservoir with the (alkaline) lysis medium connected by a first tubing and a pump and possibly a flowmeter, to the lysis unit 20 to mix the cells with the added (alkaline) lysis medium.

According to another embodiment of the present invention and as presented in the figures, the preparation unit 1 is advantageously made of two separate tanks (2 and 3), both maintained at room temperature, the first tank 2 containing NaOH (RM2-A) and the second tank 3 containing one or more detergents (RM2-B), preferably Sodium Dodecyl Sulfate (SDS).

Both tanks 2 and 3 are connected respectively by a first set of tubings 4 and 5, preferably via a first pump 6 and a second pump 7, wherein these pumps (6 and 7) are controlling the adequate added amounts of each element (NaOH, detergent and possibly one or more other molecule(s)) to form the lysis solution.

The tubings of the first tubings set (4 and 5) meet in a "Y type" connection and are pushed via an inlet or opening 8 into a first static mixer 9 used for the efficient mixing of all the introduced compounds to form the lysis buffer (RM2 buffer medium). This continuous mixing insures the stability and constant composition quality of the (alkaline) lysis medium.

The outlet or exit 10 of this first static mixer 9 is connected, via a second tubing 11 of the preparation unit 1 to the inlet or opening 21 of a second static mixer 22 which is part of the cells lysis unit 20. Preferably, the second static mixer 22 of the cells lysis unit 20 comprises between 12 and 24 mixing elements, preferably being 18 mixing elements.

The cells lysis unit 20 is the passageway described above and preferably includes a second static mixer 22, connected by a "Y-Type" connection at its inlet 21, to both the first tubing 11 of the preparation unit 1 and, via a third tubing 23 and a pump 24, to a cells suspension tank 25 containing at a suitable temperature, preferably at a temperature comprised between about 2°C and about 8°C, the suspended cells with the extra-chromosomal nucleic acid sequence(s) to recover.

Consequently, the second static mixer 22 allows an efficient mixing of the suspended cells with the lysis buffer to generate an efficient lysis of these cells. The lysis or the cells disintegration is performed by addition of the cells lysis medium, being preferably a mixture of NaOH + SDS. Although the generated solution of disintegrated cells is viscous, an efficient homogenization was obtained without degrading the plasmid.

The inventors have further optimized the tubing lengths and the pump output in order to assess the optimal mean contact time of the cells and the lysis buffer. They found that the apparatus they developed allows short duration, such as less than 5 minutes, which advantageously prevents the degradation of the extra-chromosomal nucleic acid sequence(s) of interest, and observed an optimum time of (about) 1 minute to (about) 5 minutes, more preferably (about) 2 minutes.

The outlet or exit 26 of this second static mixer 22 is connected to the features of the neutralization unit 30, via a fourth tubing 27 having an adequate length allowing the contact time.

The neutralization unit 30 comprises a neutralization tank 31 containing a neutralization medium (RM3), preferably made of a solution of acetic acid and acetate, preferably maintained at a pH comprised between 5.0 and 6.0 preferably of about 5.5 and consisting of 3M of acetate and 15% (v:v) of acetic acid, preferably used at a temperature comprised between 2°C and 8°C, preferably of about 4°C.

This neutralization tank 31 is connected, via a fifth tubing 32 and via a pump 33, to an inlet or opening 34 of a third static mixer 35. The neutralization medium can be cooled before use by storage of the tank in a cold room for a sufficient amount of time, or cooled down in a continuous manner from room temperature (comprised between 20°C and 25°C) to a lower temperature comprised between 2°C and 8°C, preferably of about 4°C, by a cryostat 36 and heat exchanger 39 surrounding a section of the fifth tubing 32.

The inlet or opening 34 of this third static mixer 35 is also connected to the fourth tubing 27 collecting the fluid with cells under lysis obtained from the second static mixer 22, but this fourth tubing 27 has an adequate length to perform a suitable lysis of the treated cells and obtain a cells lysate. Preferably, this third static mixer 35 comprises between 6 and 16 mixing elements, preferably 10 mixing elements.

The inlet 34 of this third static mixer 35 is preferably connected by a "Y-type" connection to two tubings 32 and 27, so that both fluids flows are sent to the static mixer 35 to obtain a rapid and efficient neutralization of the cells lysate to stop the lysis reaction and to form a neutralized cells lysate or cells lysate mixture.

The inventors have further optimized the tubing lengths and the pump output in order to assess the optimal mean contact time of the lysed cells under neutralization. They found that the apparatus they developed allows short duration, such as less than 3 minutes, which advantageously leads to an efficient neutralization of the lysed cells before the addition of the precipitation agent, and observed an optimum time of (about) 0.5 minute to (about) 3 minutes, more preferably (about) 1 minute.

The outlet or exit 37 of this third static mixer 35 is connected to a sixth tubing 38 collecting the obtained neutralized lysate mixture or cells lysate mixture. The sixth tubing 38 can be directed to the inlet or opening 45 of a fourth static mixer 41 forming the precipitation unit 40.

The inlet or opening 45 of the fourth static mixer 41, is also connected to a seventh tubing 42, linked via a pump 43, to a tank 44 containing a solution of divalent ions salts (RM4), preferably containing hydrated calcium chloride.

Again, a "Y-type" connection is preferably done between both tubings 38 and 42, so that both fluids flows are sent to the fourth static mixer to obtain a rapid and efficient mixing. A solution of CaCl₂.5H₂O (5M) obtained from the tank 44, via the seventh tubing 42 is added continuously into the fourth static mixer 41 to the neutralized cells lysate (or cells lysate mixture) collected from the sixth tubing 38. This fourth static mixer 41 insures a thorough mixing of the dense and viscous solutions together and improves the precipitation reaction.

This fourth static mixer 41 comprises between 4 mixing elements and 18 mixing elements, preferably only 4 mixing elements. An optimum number of 10 mixing elements could be selected for an efficient mixing of the salts with the cells lysate mixture. However, as a higher number of mixing elements will produce smaller particles that are not easily eliminated during decantation and would reduce the production and purification yield and time, the number of efficient mixing elements was reduced to 4 mixing elements.

The outlet or exit 46 of the fourth static mixer 41 is connected, via an eighth tubing 47 to a decantation tank 51 collecting the cells lysate mixture 52 obtained from the fourth static mixer 41. It will be convenient to note that the fourth static mixer 41 is optional, and tubing 38 can be directly fed to decantation tank 51.

In the apparatus according to the invention, the decantation unit 50 is used for improving decantation of a cells lysate mixture 52 from its impurities or contaminants. This decantation unit 50 comprises at least one decantation tank 51, comprising means used for improving the yield, especially the delay and the efficiency of decantation and recovery of the extra-chromosomal nucleic acid sequences of interest to be recovered and purified from this cells lysate mixture 52.

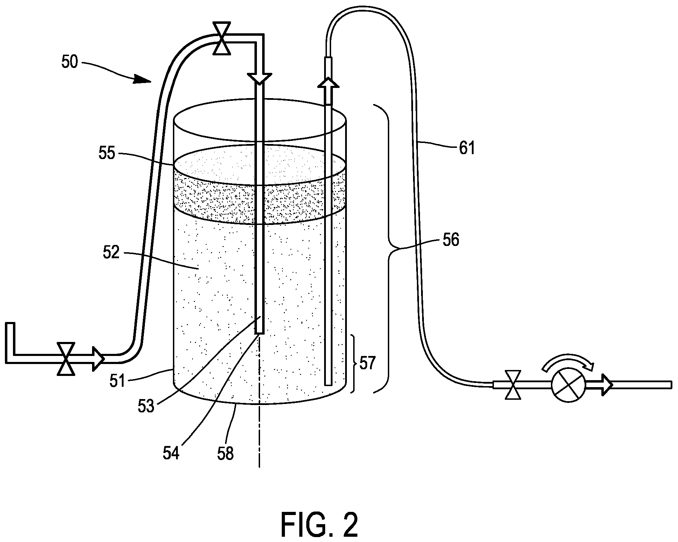

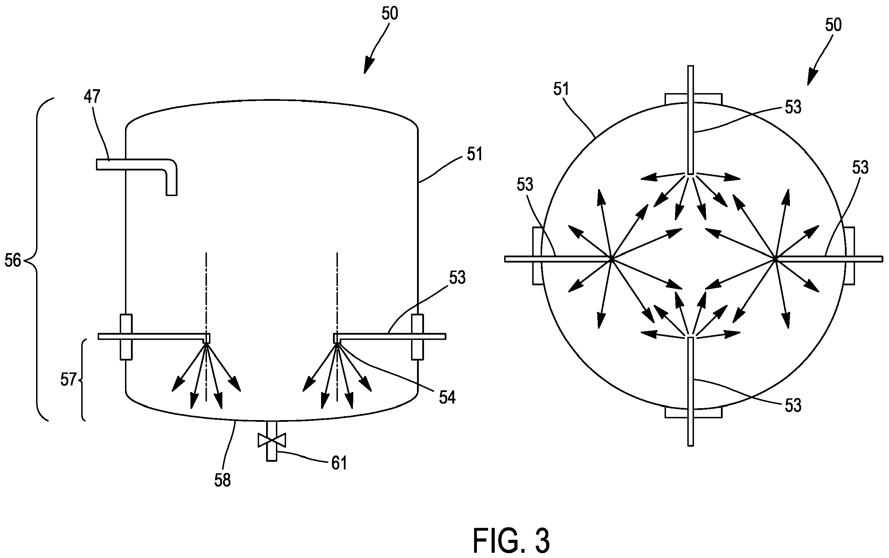

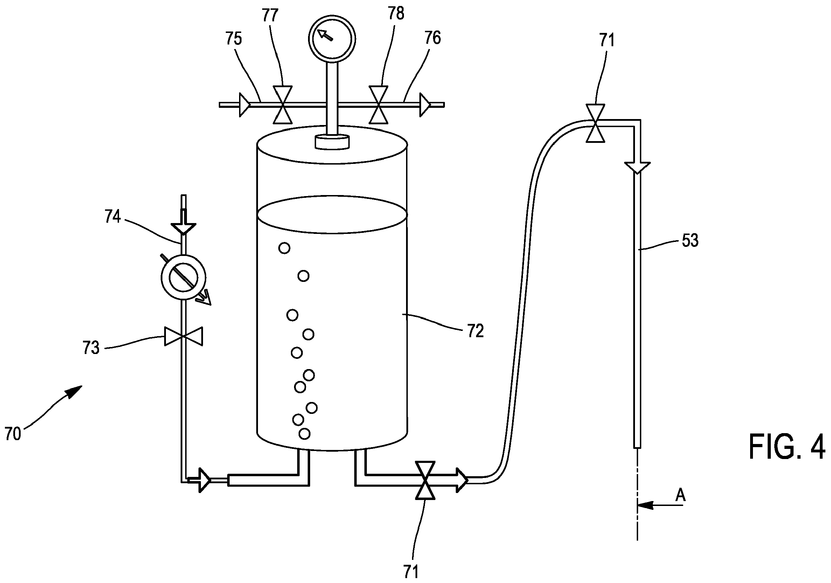

The decantation unit 50 represented in Figures 2 and 3 is connected to a floatation unit 70 (Figure 4) allowing injection inside the decantation tank 51, of an aqueous medium more preferably water, comprising large amounts of a dissolved gas preferably air, nitrogen or CO₂, more preferably air and maintained at room temperature with the gas, preferably air, introduced at a suitable pressure, preferably a pressure of at least 2 barg or higher.

The decantation is efficiently improved through an injection of the aqueous medium, preferably water comprising saturated or near-saturated levels of a dissolved gas, preferably selected from the group consisting of air, CO₂ or nitrogen, or a mixture thereof, through one or more injection pipe(s) 53 having an exit 54, eventually with a nozzle allowing injection of the gasified aqueous medium into the decantation tank 51, and therefore into the cells lysate mixture 52 present in the decantation tank 51. The exit 54 of this injection pipe 53 is disposed in the opposite direction to the surface 55 of the cells lysate mixture 52 present in the decantation tank 51 and towards to or in the direction of the bottom surface 58 of the decantation tank 51.

As represented in figure 3, the configuration of the decantation unit 50 may include a decantation tank 51 presenting a specific design, wherein more than one injection pipe(s) 53 are introduced, preferably two, three or four injection pipes 53 are introduced, possibly arranged at diagonally opposite positions in the tank 51, preferably with an exit 54 of each injection pipe 53 being disposed in the same horizontal plane advantageously at equivalent distance from each other and from the sides and bottom of the decantation tank. In Fig. 3 four injection pipes 53 are represented at diametrically or diagonally opposite positions, even though more or less injection pipes may be provided.

As represented in the figure 3, the exit 54 of each injection pipe 53 is disposed facing away from the top surface 55 of the cells lysate mixture 52 present in the decantation tank 51. Exit 54 is facing the bottom surface 58 of the tank 51 and oriented to inject the aqueous medium in tank 51 vertically downwards in the direction of this bottom surface 58 of the decantation tank 51.

In the decantation unit 50 of the apparatus according to the invention, the cells lysate mixture 52 is introduced by the eighth tubing 47 disposed along the side wall of the decantation tank 51 and fills partially the decantation tank 51. There should remain a free volume in the tank sufficient to handle the addition of the aqueous medium saturated with gas under pressure.

The exit 54 of the injection pipe(s) 53 is advantageously positioned at a height 57 from the bottom of the tank between 1/6 and 5/6 of the height 56 of the tank 51, more advantageously positioned at a height 57 from the bottom of the tank between 1/4 and 3/4 , advantageously between 1/4 and 2/4 of the tank height 56.

The injection pipe 53 may present a nozzle exit, with a narrow exit. Advantageously the injection pipe 53 presents a ratio between its internal diameter and the diameter of its nozzle exit 54, which is higher than or equal to (about) 2, more preferably higher than or equal to (about) 3, and may be lower than or equal to 50, more preferably lower than or equal to 10. This configuration and ratio between the internal diameter pipe size and its nozzle exit are useful for obtaining that depressurization of the aqueous medium starts only at the nozzle itself. By so doing, the aqueous medium is kept under a suitable elevated pressure until the exit nozzle, so that pressure falls at the nozzle exit. As a result, a rapid and efficient clarification and decantation of the cells lysate mixture 52 from its contaminants or impurities, which are dispersed in the decantation tank 51 can be obtained. The contaminants or impurities will float at the top surface 55 of the cells lysate mixture 52. Preferably this decantation duration is between 5 minutes and 3 hours but could be applied for longer periods of up to 1 or 2 days. However, in specific configuration of the apparatus of the invention allowing production of lower amounts of extrachromosomal nucleic acid sequences of interest, this ratio is of (about) 1 or near the value of 1 with an injection pipe 53 that does not include a nozzle and a reduction of the exit 54 diameter.

As represented in figure 4, the added aqueous medium containing dissolved gas is obtained from the injection pipe 53 which is connected, preferably through a valve 71 to the injection or floatation unit 70.

The injection or floatation unit 70 comprises a tank 72 containing an aqueous medium, preferably water. This aqueous medium tank 72 comprises an inlet, advantageously arranged in a lower part of tank 72, which is connected, preferably via a valve 73 to a tubing element 74 supplying in the aqueous medium tank 72 a gas, preferably air. Additional tubing elements (75 and 76) and valves (77 and 78) for a gas (air) introduction and expulsion can be connected to tank 72.

The tank 72 is advantageously a closed vessel arranged to hold a liquid under suitable elevated pressure. Tank 51 is advantageously configured to hold the cells lysate mixture 52 at substantially atmospheric pressure.

The aqueous medium is enriched and advantageously saturated with dissolved gas under pressure by a bubbling of the pressurized gas in the medium. By so doing, the aqueous medium is enriched in the dissolved gas (Henry's law). Advantageously, the bubbling can be performed for a sufficient amount of time to reach the complete saturation. The pressure in the tank 72 is advantageously kept constant during the bubbling, at a value of 2 barg or more, preferably between 2 barg and 50 barg, more preferably between 3 barg and 25 barg.

The aqueous medium enriched in dissolved gas is injected in the decantation tank 51 advantageously during a period comprised between a few seconds and a few minutes, preferably between 5 seconds and 5 minutes. Preferably, in the method and apparatus according to the invention, the volume % of the injected aqueous medium is comprised between 0.2% and 25 % (v/v), preferably comprised between 0.5% and 15%, more preferably comprised between 1% and 10%, compared to the volume of the decanted cells lysate mixture.

Referring again to Fig. 2 and 3, the decantation tank 51, further includes a collecting tubing 61 for recovering a soluble fraction or clarified phase containing the extra-chromosomal nucleic acid sequence(s), from the cells lysate mixture 52 present in the decantation tank 51.

The inlet of the collecting tubing 61 is advantageously disposed at the bottom of the decantation tank 51 for obtaining an efficient pumping of the soluble fraction or clarified phase with minimising liquid movements in the decantation tank 51 to avoid a resuspension of the contaminants or impurities precipitates. Tubing 61 advantageously removes the soluble fraction from tank 51 in the bottom part 57, below the level of exit 54 of injection pipe 53. Tubing 61 can be connected to a filtration unit.

A further purification with known means (such as filters, chromatographic columns and collecting tanks) and method steps well known by the skilled person is also obtained.

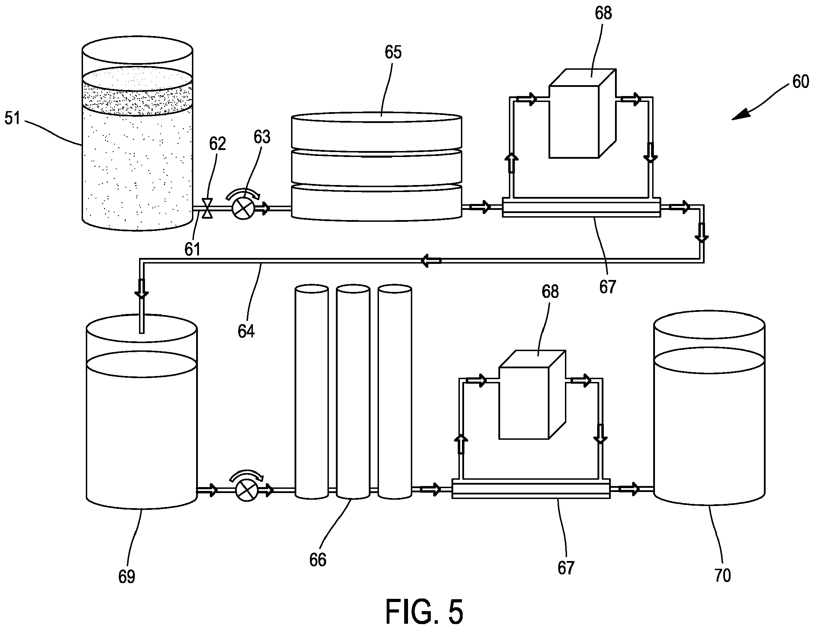

As represented in Figure 5, the flow of this soluble fraction or clarified phase of the cells lysate mixture, outside the decantation tank 51 is advantageously obtained through the opening of a valve 62 and a pump 63 connected to another section of a tubing element 64 allowing the flow of the soluble fraction or clarified phase of the cells lysate mixture towards another filtration unit 60 for its further purification with methods steps well known by the skilled person. This filtration unit can comprise one or more filters 65 and 66 and/or one or more chromatographic columns with suitable elution material. Furthermore during filtration, ultrafiltration and/or diafiltration, one or more tubing section(s), especially section(s) of the tubing element 64 could be surrounded by a heat exchange device 67 connected to a cryostat 68 for a cooling of the filtrated and clarified lysate to a temperature as closed to 4°C as possible (in the rage of 2°C to 8°C) that can be kept for an adequate time (overnight) in suitable (and possibly refrigerated) tanks 69 and 70.

In the apparatus and method according to the invention, the tubings may include flow meters or other devices for controlling the introduction of suitable volumes (per unit of time) of the different fluids necessary for the lysis system, the tanks or reservoirs. The apparatus may also include weight monitoring elements, such as weight balances of the different tanks or reservoirs to measure the amount of introduced solution or active compounds (lysis solution, neutralization solution, cells in suspension, aqueous medium saturated in gas under pressure,...) at each step of the method according to the invention. Conditions and efficiency of purification obtained by the apparatus and the method of the invention are summarized in the following table 1.

**TABLE 1: overview of possible process conditions**

| | |
|---|---|
| Aqueous medium temperature | 4°C to room temperature (the air dissolves much slower at 4 °C) |
| Air pressure | 6 barg |
| Compressed gas bubbling in the aqueous medium duration | >= 10 minutes |
| Lysate volume treated | From almost nothing (less than 1 liter) up to cubic meters (3 x 1500 L planned for the Step2 capacity expansion at KEGT), depending on the injection system. |
| Aqueous medium volume (% v/v) | From 0.5 to 20 % per injection, successive injections improving the floatation effect |
| Aqueous medium injection duration | From 5 seconds to 5 minutes depending on the injection flow rate and injected volume. |
| Floatation duration | Between 5 min and 3 hours, typically 15-30 min between each injection or before collection. |
| Full process duration | From cells lysis to final 0.2 µm filtration: **less than around 8 hours at any scale.** |
| Decanting efficiency | Volume of clear lysate recovered / total volume (lysate + precipitates layer): By gravimetric decanting: 71 % to 77 % recovery |
| | With floatation: 85 % to 88 % recovery |
| | ⇒ **10 to 15% volume recovery gain** |
| Turbidimetry reduction | By gravimetric decanting: about 50 NTU |
| | With floatation: < 20 NTU |
| | ⇒ **More than 2x turbidimetry reduction** |
| | ⇒ **Depth filtration membrane area needed decreased by about the same factor.** |

The advantages brought by the new process and apparatus of the invention are numerous. It is a simple and robust, efficient, reproducible and automatable process and apparatus or plant.

A first goal of the new process and apparatus of the invention was to allow the treatment of large amounts of cells pellets by increasing the flow rates, while keeping the same process duration time (lower than about 4 hours) for every scale. This goal was reached thanks to the use of static mixers, which also allow a reduction of the number of batches necessary and an easy follow up of each step. The new large scale system for the Step 2 capacity expansion at KEGT (3 x 1500 L) would never have been possible without this newly developed process and apparatus.

A second goal of the process and apparatus according to the invention was to keep the decantation duration at a maximum of two hours. This goal was reached and even exceeded, the full decantation being obtained after only about 1 hour or even lower.

A third goal was to have a robust decantation where, for each run, almost no particles were left on the bottom of the tank or dispersed in the cells lysate mixture, as they quickly clog the clarification depth filters and should not be pumped. This goal was reached as almost all particles are floating on the top of the tank after the floatation, and even exceeded as the clarified fraction of the lysate is much clearer than with a classical gravimetric decanting (decreased turbidity). As a consequence, less depth filters surface is needed for the clarification, which means lower costs, wastes, space needed in the zone, handling, etc.

A last goal was to enhance the recovery of the lysate, and was also reached as the lysate lost in the precipitates layer is now replaced by gas, this precipitates layer floating above the cells lysis mixture instead of being immersed in it. The volume of recovered lysate has therefore been increased by 10 % to 15%.

## Claims

1. A method for obtaining an extra-chromosomal nucleic acid sequence from cells and comprising the steps of:
a) optionally cultivating cells comprising an extra chromosomal nucleic acid sequence of interest;
b) disintegrating the cells by mixing the cells with a lysis medium to form lysed cells;
c) neutralizing the lysed cells with a neutralization solution, preferably a neutralization solution composed of an acetic acid/acetate solution, to produce a cells lysate mixture formed by a soluble fraction and a precipitate;
d) optionally precipitating further contaminants of the extra-chromosomal nucleic acid sequence of interest, by mixing a solution containing one or more salts to the cells lysate mixture, wherein the salt(s) are selected from the group consisting of CaCl₂, MgCl₂, ZnCl₂, SrCl₂, BaCl₂, LiCl, ammonium acetate, ammonium sulfate, sodium sulfate and magnesium sulfate or a mixture thereof;
e) separating the soluble fraction comprising the extra-chromosomal nucleic acid sequence of interest from the cells lysate mixture; and
f) recovering the separated soluble fraction comprising the extra-chromosomal nucleic acid sequence of interest,
wherein the separating step e) comprises dissolving a gas in an aqueous medium under a pressure higher than atmospheric pressure followed by injecting the aqueous medium into the cells lysate mixture.

2. The method of claim 1, wherein the gas is dissolved in the aqueous medium by gas bubbling under pressure of 2 barg or higher, prior to injecting the aqueous medium in the cells lysate mixture.

3. The method of claim 1 or 2 wherein the aqueous medium is injected in the cells lysate mixture in a volume amount comprised between 0.2% and 25%, preferably between 0.5% and 15%, more preferably between 1% and 10% V/V, relative to the volume of the cells lysate mixture.

4. The method of any one of the preceding claims, wherein the aqueous medium is injected under a pressure of at least 2 barg in the cells lysate mixture, the cells lysate mixture being at atmospheric pressure.

5. The method of any one of the preceding claims, wherein the aqueous medium injected in the cells lysate mixture is saturated with the gas.

6. The method according to any one of the preceding claims, wherein the mixing of the cells with the lysis medium (step b) is done in a static mixer (22), preferably a static mixer (22) comprising 6 or more mixing elements and wherein a mixing linear speed of the introduced mixture of cells and lysis medium into the static mixer (22) is preferably equal or higher than 150 cm/min.

7. The method of any one of the preceding claims, wherein the aqueous medium is injected intermittently in the cells lysate mixture.

8. The method according to any one of the preceding claims, wherein neutralizing the lysed cells with a neutralization solution in step (c) is done in a static mixer (35), preferably a static mixer comprising 4 or more mixing elements and wherein the mixing linear speed of the mixture of lysed cells and neutralization solution in the static mixer (35) is equal or higher than 100 cm/min.

9. The method according to any one of the preceding claims, wherein the cells lysate mixture (52) is contained in a tank (51) and the aqueous medium is injected downwards towards a bottom (58) of the tank.

10. An apparatus for performing the method according to any of the preceding claims, comprising:
- a preparation unit (1) comprising one or more tanks (2, 3) containing the constituents of a lysis medium and an outlet (10),
- a cells lysis unit (20) comprising a cells suspension tank (25) containing cells with the extra-chromosomal nucleic acid sequence of interest, and further comprising an inlet (21) and an outlet (26), wherein the inlet (21) is fluidly connected to the cells suspension tank and with the outlet (10) of the preparation unit (1),
- a neutralization unit (30) for generating a cells lysate mixture, comprising a neutralization medium tank (31), an inlet (34) and an outlet (37), wherein the inlet (34) is in fluid connection with the neutralization medium tank (31) and with the outlet (26) of the cells lysis unit (20),
- a separation unit (50) comprising a separation tank (51) having an inlet fluidly connected to the outlet (37) of the neutralization unit (30) for receiving the cells lysate mixture, and
- an injection unit (70) comprising means for dissolving a gas in the aqueous medium at a pressure higher than atmospheric pressure and means (54) for injecting the aqueous medium with the dissolved gas in the separation tank (51).

11. The apparatus of claim 10, wherein the means for injecting the aqueous medium with the dissolved gas comprise at least one injection pipe (53) having an exit (54) disposed towards a bottom surface (58) of the separation tank (51), preferably an injection pipe (53) comprising an exit nozzle (54) and wherein the ratio between the injection pipe (53) internal diameter and its exit nozzle (54) diameter is at least 2.

12. The apparatus of claim 10 or 11, wherein the means for injecting the aqueous medium is disposed to inject the aqueous medium in the separation tank (51) at a height (57) from the bottom of the tank (58) comprised between 1/6 and 5/6 of the total height (56) of the separation tank, preferably at a height from the bottom of the tank (58) comprised between 1/4 and 2/4 of the total height.

13. The apparatus according to any one of the claims 10 to 12, wherein the means for dissolving the gas in the aqueous medium comprises an aqueous medium tank (72) and means (74) for bubbling the gas through the aqueous medium tank (72), the means for bubbling being configured to inject the gas in the aqueous medium tank (72) at a preferred pressure higher than or equal to 2 barg, preferably between 2 barg and 50 barg, more preferably between 3 barg and 25 barg.

14. The apparatus according to claim 13, wherein the means for dissolving the gas is configured to put the aqueous medium tank under a pressure of at least 2 barg.

15. The apparatus according to any one of the claims 10 to 14, wherein the injection unit is configured to inject the aqueous medium with the dissolved gas intermittently in the separation tank.
